# EUROPÄISCHE PATENTANMELDUNG

(11) **EP 3 654 034 A1**
(43) Veröffentlichungstag der Anmeldung: **20.05.2020**
(21) Anmeldenummer: 19209815.0
(22) Anmeldetag: 18.11.2019
(51) Int. Cl.: G01N 33/36, G01B 11/25

(54) **VERFAHREN ZUM KALIBRIEREN SOWIE VERFAHREN UND VORRICHTUNG ZUR VERMESSUNG**

(30) Priorität: 19.11.2018 DE 102018128990
(71) Anmelder: Deutsches Zentrum für Luft- und Raumfahrt e.V., 51147 Köln (DE)
(72) Erfinder: Meister, Sebastian, 21680 Stade (DE)
(74) Vertreter: Gramm, Lins & Partner Patent- und Rechtsanwälte PartGmbB

(57) **Zusammenfassung**

Die Erfindung betrifft ein Verfahren zum Kalibrieren einer Sensoranordnung, die zum Ermitteln von fusionierten Messdaten einer Fasermaterialoberfläche eines Fasermaterial eines Faserverbundwerkstoffes, aus dem ein Faserverbundbauteil herstellbar ist, vorgesehen ist, wobei die Sensoranordnung einen ersten Sensor einer ersten Sensorart zum Erfassen einer ersten Merkmalsart der Fasermaterialoberfläche und wenigstens einen zweiten Sensor einer zweiten Sensorart zum Erfassen einer von der ersten Merkmalsart verschiedenen zweiten Merkmalsart der Fasermaterialoberfläche hat, wobei das Verfahren die folgenden Schritte umfasst:
- Bereitstellen eines Kalibrierkörpers, der Merkmale der ersten Merkmalsart und Merkmale der zweiten Merkmalsart aufweist;
- Ermitteln von ersten Messdaten durch Vermessen des Kalibrierkörpers mittels des ersten Sensors der Sensoranordnung und Ermitteln von zweiten Messdaten durch Vermessen des Kalibrierkörpers mittels des zweiten Sensors der Sensoranordnung;
- Erkennen von ersten Merkmalen der ersten Merkmalsart aus den ersten Messdaten und Erkennen von zweiten Merkmalen der zweiten Merkmalsart aus den zweiten Messdaten mittels einer Auswerteeinheit;
- Vergleich der erkannten ersten Merkmale mit den erkannten zweiten Merkmalen bezogen auf die relative Position, Orientierung und/oder Form der Merkmale; und
- Ermitteln einer Übertragungsfunktion in Abhängigkeit von dem Vergleich der erkannten Merkmale, um einen jeweiligen Messwert der ersten Messdaten mit einem entsprechenden Messwert der zweiten Messdaten zu korrelieren.

## Beschreibung

Die vorliegende Erfindung betrifft ein Verfahren zum Kalibrieren einer Sensoranordnung, die zum Ermitteln von fusionierten Messdaten einer Fasermaterialoberfläche eines Fasermaterials eines Faserverbundwerkstoffes, aus dem ein Faserverbundbauteil herstellbar ist, vorgesehen ist. Die Erfindung betrifft ebenso ein Verfahren zum Vermessen einer derartigen Fasermaterialoberfläche sowie eine Vorrichtung hierzu mit entsprechend kalibrierten Sensoren.

Auf Grund der gewichtspezifischen Festigkeit und Steifigkeit von Faserverbundbauteilen, die aus Faserverbundwerkstoffen hergestellt sind, sind derartige Bauteile aus der Luft- und Raumfahrt sowie aus dem Automobilbereich heutzutage kaum mehr weg zu denken. Bei der Herstellung eines Faserverbundbauteils wird dabei ein in ein Fasermaterial infundiertes Matrixmaterial meist unter Temperatur- und Druckbeaufschlagung ausgehärtet und bildet so nach dem Aushärten eine integrale Einheit mit dem Fasermaterial. Die Verstärkungsfasern des Fasermaterials werden hierdurch in vorgegebene Richtung gezwungen und können so die auftretenden Lasten in die vorgegebene Richtung abtragen.

Faserverbundwerkstoffe, aus denen derartige Faserverbundbauteile hergestellt werden, weisen in der Regel zwei Hauptbestandteile auf, nämlich zum einen ein Fasermaterial und zum anderen ein Matrixmaterial. Hierneben können noch weitere sekundäre Bestandteile verwendet werden, wie bspw. Bindermaterialien oder zusätzliche Funktionselemente, die in das Bauteil integriert werden sollen. Werden für die Herstellung trockene Fasermaterialien bereitgestellt, so wird während des Herstellungsprozesses das Matrixmaterial des Faserverbundwerkstoffes in das Fasermaterial durch einen Infusionsprozess infundiert, durch den das trockene Fasermaterial mit dem Matrixmaterial benetzt wird. Hierneben sind auch Faserverbundwerkstoffe bekannt, bei denen das Fasermaterial mit dem Matrixmaterial bereits vorimprägniert ist (sogenannte Prepregs).

Vor dem Aushärten des Matrixmaterials wird in der Regel das Fasermaterial in ein Formwerkzeug eingebracht, dass mit seiner formgebenden Werkzeugoberfläche die spätere Bauteilform nachbildet. Dabei können sowohl trockene als auch vorimprägnierte Fasermaterialien in das Formwerkzeug abgelegt werden. Zur Herstellung von großskaligen Strukturbauteilen, wie bspw. Flügelschalen von Verkehrsflugzeugen, werden zur Optimierung des Ablegeverfahrens automatisierte Faserlegeprozesse verwendet, bei denen mit Hilfe einer Fertigungsanlage und mindestens einem Faserlegekopf einem dem Faserlegekopf zugeführtes quasi endloses Fasermaterial auf das Werkzeug gedrückt und somit abgelegt wird. Bei der sogenannten Fiber Placement Technologie werden bspw. vorimprägnierte Fasermaterialien bahnweise auf dem Formwerkzeug mit Hilfe eines solchen Faserlegekopfes abgelegt. Der Faserlegekopf ist dabei an einem Roboter angeordnet und kann gegenüber dem Formwerkzeug relativ bewegt werden. Hierdurch können die einzelnen Faserbahnen auf der Werkzeugoberfläche schließlich abgelegt werden. Bei der Fiber Placement Technologie werden gleichzeitig mehrere, meistens 8, 16 oder 32 schmale Materialstreifen, sogenannte Tows, auf dem Formwerkzeug abgelegt. Bei der Fiber Tape Laying Technologie werden meist breite Faserbahnen (auch Tapes genannt), in der Regel 150 mm, 300mm oder 600mm breit, mit einer Dicke von wenigen Zehntel Millimeter mit Hilfe des Faserlegkopfes auf dem Formwerkzeug abgelegt.

Aus der DE 10 2010 015 027 B1 ist beispielhaft eine solche Faserlegeanlage bekannt, bei der mehrere Roboter auf einem Schienensystem um ein in der Mitte angeordnetes Formwerkzeug herum bewegbar sind und dabei mit Hilfe von Faserlegeköpfen das Fasermaterial auf dem Werkzeug simultan ablegen können.

Mit Hilfe der Faserlegeköpfe und den Fasermaterialien werden dann in dem Formwerkzeug Faserlaminate, auch Faserpreformen genannt, hergestellt, bei denen schlussendlich der Prozess des Infundierens des Matrixmaterials bzw. des Aushärtens des Matrixmaterials noch nicht stattgefunden hat. Derartige Faserlaminate bilden somit das Bauteil in seinem Rohzustand vor dem Aushärten des Matrixmaterials. Faserlaminate, gerade bei großskaligen Strukturbauteilen, bestehen dabei nicht selten aus mehreren Einzellagen, die je nach Anwendungsgebiet des Bauteils auch mehr als 100 Lagen umfassen können. Zur Gewährleistung einer hohen Bauteilqualität muss die Anzahl auftretender Prozessfehler bei der Ablage des Fasermaterials (bspw. Tapes oder Tows) minimiert bzw. gänzlich verhindert werden. Das Auftreten von Prozessfehlern bzw. Fertigungsabweichungen mit großem negativen Einfluss auf die Festigkeit der Bauteilstruktur, muss nach Möglichkeit vollständig im Prozess erkannt und wünschenswerterweise auch unterbunden werden.

Aus der DE 10 2013 112 260 A1, DE 10 2013 104 545 A1 sowie DE 10 2013 104 546 A1, die aus dem Hause des Anmelders stammen, werden Verfahren zum Erkennen von Fehlstellen von auf einer Werkzeugoberfläche abgelegter Faserhalbzeuge bzw. Fasermaterialien offenbart, wobei mittels eines optischen Lichtprojektionsverfahrens, bei dem das abgelegte Fasermaterial mit einer Lichtquelle aus einem ersten Winkel beleuchtet und dann das von der Faseroberfläche reflektierte Licht aus einem anderen Winkel aufgenommen wird, ein Höhenprofil der Faserhalbzeugoberfläche erstellt wird. Mit Hilfe einer Bildauswerteeinheit wird dann das erstellte Höhenprofil untersucht und analysiert, um so Fehlstellen in den abgelegten Fasermaterialien detektieren zu können. Dabei kann u. a. vorgesehen sein, dass das Ermitteln des Höhenprofils mit Hilfe des Lichtschnittverfahrens im Nachgang zum Ablegeprozess stattfindet, sodass Fertigungsabweichungen gerade abgelegter Fasermaterialien bzw. Fasermaterialabschnitte sofort erkannt werden können. Hierdurch wird eine messtechnische Erfassung von Fertigungsabweichungen, wie bspw. Ablegefehler, im Nachlauf des Ablegeprozesses möglich, um einen nachgelagertes Qualitätssicherungssystem zu erhalten. So können Fehler erkannt, und gegebenenfalls ausgebesset werden. Außerdem ist eine Dokumentation der Bauteilherstellung möglich.

Nicht selten werden zum Vermessen von Fasermaterialoberflächen entsprechende Sensoranordnungen bestehend aus mehreren Sensoren verwendet, wobei die verwendeten Sensoren jeweils unterschiedlicher Sensorarten entstammen. So können bei der Verwendung von optischen Sensoren unterschiedliche Sensorarten verwendet werden, wie bspw. Zeilensensoren (Lichtschnittsensoren), 2D-Flächensensoren, wie bspw. 2D-Kamerasysteme, Thermographie-Kamerasysteme und dergleichen oder 3D-Kamerasysteme, die zwei oder mehr Bildsensoren beabstandet voneinander aufweisen, und so einen 3D-Datensatz erzeugen können. Durch den parallelen Einsatz dieser verschiedenen Sensorarten und einer entsprechenden Datenfusion lassen sich Fehlstellen auf der Fasermaterialoberfläche deutlich prozesssicherer erkennen, sodass dem sehr früh entgegengewirkt werden kann.

Ein Problem hierbei ist jedoch, dass für eine Sensordatenfusion verschiedener Sensorarten eine Kalibrierung gewährleistet werden muss, die sicherstellt, dass jedem Messwert des ersten Datensatzes auch ein entsprechender richtiger Messwert dem zweiten Datensatz zugeordnet wird. Andernfalls referenzieren die gewonnenen Messdaten der verschiedenen Sensorarten auf unterschiedlichen Bauteilpositionen und können nicht sinnvoll miteinander fusioniert werden. Hierbei muss berücksichtigt werden, dass die Sensoranordnung gegenüber der Fasermaterialoberfläche beweglich ausgebildet sein kann und insbesondere ihre Position, Orientierung und Neigung gegenüber der Fasermaterialoberfläche im Scanprozess verändern kann.

Es ist demzufolge Aufgabe der vorliegenden Erfindung ein verbessertes Verfahren zum Kalibrieren einer solchen Sensoranordnung anzugeben, um eine Sensoranordnung mit zwei oder mehr verschiedenen Sensorarten zum Zwecke einer Datenfusion verwenden zu können.

Die Aufgabe wird mit dem Verfahren gem. Anspruch 1 erfindungsgemäß gelöst.

Gemäß Anspruch 1 wird ein Verfahren zum Kalibrieren einer Sensoranordnung vorgeschlagen, wobei die Sensoranordnung zum Ermitteln von fusionierten Messdaten einer Fasermaterialoberfläche eines Fasermaterials eines Faserverbundwerkstoffes ausgebildet ist. Der Faserverbundwerkstoff weist dabei gattungsgemäß ein Fasermaterial und ein Matrixmaterial auf, dass gegebenenfalls schon in das Fasermaterial vorinjiziert ist (sogenannte Prepregs). Aus dem Faserverbundwerkstoff soll dann später ein Faserverbundbauteil, insbesondere ein großskaliges Faserverbundbauteil hergestellt werden. Das hierfür verwendete Fasermaterial des Faserverbundwerkstoffes wird zu diesem Zwecke auf ein Formwerkzeug abgelegt, wobei das Formwerkzeug der späteren Bauteilform entspricht. Während oder nach dem Ablegen des Fasermaterials wird nun mit Hilfe der Sensoranordnung die Fasermaterialoberfläche der abgelegten Fasermaterialien erfasst und gegebenenfalls entsprechend ausgewertet, um so Fehlstellen erkennen zu können.

Die Sensoranordnung weist dabei einen ersten Sensor einer ersten Sensorart zum Erfassen einer ersten Merkmalsart der Fasermaterialoberfläche und wenigstens einen zweiten Sensor einer zweiten Sensorart zum Erfassen einer von der ersten Merkmalsart verschiedenen zweiten Merkmalsart der Fasermaterialoberfläche auf, sodass mit Hilfe der Sensoranordnung verschiedene Merkmalsarten der Fasermaterialoberfläche erfasst werden können. Wie später noch gezeigt, kann bspw. eine erste Merkmalsart ein 3D-Höhenprofil sein, dass mit Thermographiedaten der Fasermaterialoberfläche fusioniert werden soll.

Erfindungsgemäß ist nun vorgesehen, dass für die Kalibrierung einer solchen Sensoranordnung zunächst ein Kalibrierkörper bereitgestellt wird, der Merkmale der ersten Merkmalsart, die von dem ersten Sensor der ersten Sensorart erfassbar sind, und Merkmale der zweiten Merkmalsart, die durch den zweiten Sensor der zweiten Sensorart erfassbar sind, aufweist. Ein solcher Kalibrierkörper weist demzufolge Merkmale auf, die zum einen von der ersten Sensorart erfasst werden können und entsprechende Merkmale, die von der zweiten Sensorart erfasst werden können.

Dieser Kalibrierkörper wird nun innerhalb eines von der Sensoranordnung erfassbaren Messbereichs angeordnet, wobei die Sensoranordnung dann diesen Kalibrierkörper vermisst. Hierbei werden erste Messdaten des Kalibrierkörpers mittels des ersten Sensors der ersten Sensorart und zweite Messdaten mittels des zweiten Sensors der zweiten Sensorart durch die Sensoranordnung ermittelt. Die ersten Messdaten enthalten demzufolge die von dem ersten Sensor aufgezeichneten ersten Merkmale des Kalibrierkörpers, während die zweiten Messdaten die durch den zweiten Sensor erfassten zweiten Merkmale des Kalibrierkörpers enthalten.

Mit Hilfe einer Auswerteeinheit werden diese in den Messdaten enthaltenen Merkmale erkannt, d. h. die ersten Merkmale der ersten Merkmalsart aus den ersten Messdaten sowie die zweiten Merkmale der zweiten Merkmalsart aus den zweiten Messdaten, wobei dann die in den Messdaten erkannten Merkmale hinsichtlich ihrer relativen Position, Orientierung und/ oder Form zueinander verglichen werden. Basierend auf dem Vergleich dieser Merkmale, d. h. ein Vergleich der erkannten ersten Merkmale mit entsprechenden erkannten zweiten Merkmalen der zweiten Merkmalsart, kann nun die Abweichung bezogen auf die Position, Orientierung und/ oder Form der ersten Merkmale von den zweiten Merkmalen (oder andersherum) festgestellt werden. Anhand dieses Vergleiches und der sich daraus ergebenden Abweichung zwischen den ersten Merkmalen und den zweiten Merkmalen wird nun eine Übertragungsfunktion ermittelt, die einem jeweiligen Messwert der ersten Messdaten mit einem entsprechenden Messwert der zweiten Messdaten korreliert. Mit Hilfe dieser Übertragungsfunktion, die sich aus dem Vergleich der Merkmale des Kalibrierkörpers ableiten lässt, können so die ersten Messdaten des ersten Sensors der ersten Sensorart in Bezug gesetzt werden zu den zweiten Messdaten des zweiten Sensors der zweiten Sensorart, wodurch die ersten Messdaten und die zweiten Messdaten zu einem späteren Zeitpunkt miteinander fusioniert werden können, um so noch präziser und prozesssicherer Fehlstellen auf der Fasermaterialoberfläche der abgelegten Fasermaterialien erkennen zu können.

Durch die Übertragungsfunktion wird somit das Messbildkoordinatensystem des ersten Sensors der ersten Sensorart in das Messbildkoordinatensystem des zweiten Sensors der zweiten Sensorart (oder andersherum) überführt, sodass durch ein quasi gemeinsames Messbildkoordinatensystem eine Sensordatenfusion ermöglicht wird. Darüber hinaus lässt sich aufgrund der starren Anordnung der Sensoranordnung an dem Bewegungsautomaten des Weiteren eine Überführung des gemeinsamen Messbildkoordinatensystems in das Roboterkoordinatensystem überführen, wodurch jeder Messpunkt auf der Fasermaterialoberfläche einer konkreten Position in den jeweiligen Messdaten zugeordnet werden kann. Dies ist besonders dann vorteilhaft, wenn die verschiedenen Sensorarten zeitlich voneinander verschiedene Erfassungsfrequenzen aufweisen und zeitlich bei der Erfassung nicht synchronisiert sind.

Die Sensorarten unterscheiden sich dabei hinsichtlich der Art der Merkmalserfassung und/oder hinsichtlich der erfassbaren Merkmale.

In einer Ausführungsform handelt es sich bei dem ersten Sensor der Sensoranordnung um einen Laserlichtschnittsensor, mit dem zeilenweise ein Höhenprofil als erste Messdaten aufgenommen wird. Ein Laserlichtschnittsensor ist dabei eine Art Zeilensensor, der eine Laserlichtlinie auf die Fasermaterialoberfläche projiziert und dann aus einem von der Projektion verschiedenen Orientierung aufnimmt. Anhand des rückprojizierten Laserlichtes kann dann ein Höhenprofil entlang der betreffenden Zeilenposition ermittelt werden. Dies ergibt sich aus der Abweichung der aufgenommenen Laserlinie von einer idealen Gerade. Die absolute Position der Laserlinie auf dem Sensor kann dabei ebenfalls relevant sein, um über eine Übertragungsfunktion die absolute metrische Distanz der betrachteten Oberfläche zum Sensor zu bestimmen.

Im Bezug hierzu wird ein Kalibrierkörper bereitgestellt, dessen Oberfläche mindestens einen Bereich, vorzugsweise mehrere Bereiche mit einem abweichenden Höhenniveau hat. Ein solcher Kalibrierkörper kann bspw. eine plane und ebene Oberfläche aufweisen, in der vorzugsweise kreisrunde Vertiefungen vorgesehen sind, die die Bereiche mit einem abweichenden Höhenniveau darstellen. Aufgrund der kreisrunden Form lassen sich diese Bereiche in einer nachgelagerten Auswertung als entsprechende Merkmale der ersten Merkmalsart identifizieren, wobei bspw. die Position dieser Merkmale (Bereiche mit abweichendem Höhenniveau) durch den Mittelpunkt als Referenzpunkt definiert wird.

In einer weiteren Ausführungsform ist der zweite Sensor der Sensoranordnung eine Thermographie-Kamera, mit dem ein flächiges, 2D-Thermographiebild als zweite Messdaten aufgenommen wird. Die Thermographie-Kamera als zweiter Sensor der zweiten Sensorart nimmt dabei digitale thermographische Bilddaten der Fasermaterialoberfläche des abgelegten Fasermaterials auf, wobei mehrere einzelne thermographische Bilder durch die Thermographie-Kamera aufgenommen werden, woraus sich dann ein gesamtes thermographisches Bild der Fasermaterialoberfläche der abgelegten Fasermaterialien ergibt. Die Thermographie-Kamera nimmt somit bevorzugter Weise nicht nur ein einziges thermographisches Bild der gesamten Fasermaterialoberfläche auf, sondern eine Vielzahl von Einzelbildern die dann zusammen ein digitales thermographisches Abbild der Fasermaterialoberfläche bilden.

Dabei ist es besonders vorteilhaft, wenn die Thermographie-Kamera als zweiter Sensor zusammen mit der Ausführungsform des Laserlichtschnittsensors als ersten Sensor kombiniert wird, sodass neben einem Höhenprofil der Fasermaterialoberfläche auch thermographische Bildinformationen der Fasermaterialoberfläche zu einem Datensatz fusioniert werden können.

Alternativ ist es auch denkbar, dass der zweite Sensor ein 2D-Flächensensor zur Aufnahme eines zweidimensionalen flächigen Digitalbildes der Fasermaterialoberfläche ist, wobei dieses zweidimensionale flächige Digitalbild dann als zweite Messdaten verwendet wird.

Bezüglich der Thermographie-Kamera ist es darüber hinaus besonders vorteilhaft, wenn der Kalibrierkörper derart bereitgestellt wird, dass an dem Kalibrierkörper mindestens ein Bereich mit einer abweichenden thermographischen Rückstrahleigenschaft vorgesehen ist. Dies kann bspw. dadurch erreicht werden, dass der Kalibrierkörper aus mehreren Einzellagen hergestellt ist, die jeweils ein unterschiedliches Rückstrahlverhalten aufweisen, sodass Bereiche mit einer hohen Rückstrahlintensität sich mit Bereichen einer niedrigeren Rückstrahlintensität auf der Oberfläche des Kalibrierkörpers abwechseln.

Die Bereiche mit der abweichenden thermographischen Rückstrahleigenschaft (also entweder die erhöhte Rückstrahleigenschaft oder die verringerte Rückstrahleigenschaft) werden dann als entsprechende zweite Merkmale der zweiten Merkmalsart aus den zweiten Messdaten (Thermographiedaten) ermittelt und können dann zur Ermittlung der Übertragungsfunktion herangezogen werden.

Ist der erste Sensor ein Laserlichtschnittsensor und der zweite Sensor eine Thermographie-Kamera, so ist es besonders vorteilhaft, wenn der Kalibrierkörper derart bereitgestellt wird, dass der mindestens eine Bereich mit dem abweichenden Höhenniveau (erstes Merkmal der ersten Merkmalsart) dem Bereich mit der mindestens einen abweichenden thermographischen Rückstrahleigenschaft (zweites Merkmal der zweiten Merkmalsart) entspricht. Hierdurch wird es möglich, die Ablage der ersten Merkmale von den zweiten Merkmalen in den jeweiligen Messdaten hinsichtlich der Position, Orientierung und Form genauestens zu bestimmen, indem mittels der Auswerteeinheit die ersten Merkmale der ersten Merkmalsart in Deckung gebracht werden mit den zweiten Merkmalen der zweiten Merkmalsart. Hierdurch ist selbstverständlich mitumfasst, dass die Positionen der beiden verschiedenen Merkmalsarten auf dem Kalibrierkörper nicht exakt übereinstimmen, sondern unter einer vorgegebenen geometrischen Beziehung füreinander abweichen, sodass unter Kenntnis der bekannten Abweichung dann ebenfalls die Merkmale in Deckung gebracht werden können.

Ein derartiger Kalibrierkörper kann dabei dergestalt sein, dass die Bereiche mit dem abweichenden Höheniveau auch gleichzeitig diejenigen Bereiche sind, die eine von dem Rest des Kalibrierkörpers verschiedene thermographische Rückstrahleigenschaft besitzt.

In einer weiteren vorteilhaften Ausführungsform werden in den ersten und/oder zweiten Messdaten zunächst intrinsische Sensorabweichungen durch die Auswerteeinheit eliminiert, die sich bspw. aus Linseneffekten und anderen Einflussfaktoren, die sich auf den Sensor direkt beziehen, beziehen.

Darüber hinaus ist es vorteilhaft, wenn die Übertragungsfunktion in Abhängigkeit von einer translatorischen Ablage der erkannten ersten Merkmale in Bezug zu den erkannten zweiten Merkmalen, in Abhängigkeit von einer rotatorischen Ablage der erkannten ersten Merkmale in Bezug zu den erkannten zweiten Merkmalen, und/ oder in Abhängigkeit von einer perspektivischen Verzehrung der erkannten ersten Merkmale in Bezug zu den erkannten zweiten Merkmalen ermittelt wird. Der Vorteil von kreisrunden Merkmalen ist, dass der Mittelpunkt eines Kreises auch bei perspektivischer Verzerrung immer an derselben Position liegt. Lediglich die Form des Merkmals ändert sich. Dadurch wird der Einfluss einer perspektivischen Verzerrung quasi eliminiert.

Damit lässt sich die Übertragungsfunktion durch entsprechende geometrische Beziehungen zwischen den ersten Messdaten und den zweiten Messdaten ermitteln und für eine Sensorfusion bereitstellen.

Ein weiterer Aspekt der vorliegenden Erfindung betrifft ein Verfahren zum Vermessen einer Fasermaterialoberfläche eines Fasermaterials eines Faserverbundwerkstoffes, aus dem ein Faserverbundbauteil herstellbar ist, mittels einer Sensoranordnung, wobei die Sensoranordnung einen ersten Sensor einer ersten Sensorart zum Erfassen einer ersten Merkmalsart der Fasermaterialoberfläche und wenigstens einem zweiten Sensor einer zweiten Sensorart zum Erfassen einer von der ersten Merkmalsart verschiedenen zweiten Merkmalsart der Fasermaterialoberfläche hat, wobei das Verfahren die folgenden Schritte umfasst:
- Kalibrieren der Sensoranordnung mit dem Verfahren wie vorstehend beschrieben;
- Vermessen der Fasermaterialoberfläche mittels der Sensoranordnung, wobei während des Vermessens erste Messdaten durch den ersten Sensor und zweite Messdaten durch den zweiten Sensor ermittelt werden und
- Fusionieren der ersten Messdaten mit den zweiten Messdaten unter Berücksichtigung der bei der Kalibrierung ermittelten Übertragungsfunktion zu einem fusionierten Messdatensatz.

Vorteilhafte Ausgestaltungen finden sich in den entsprechenden Unteransprüchen. So ist es denkbar, dass der erste Sensor der Sensoranordnung ein Laserlichtschnittsensor ist, mit dem zeilenweise ein Höhenprofil als erste Messdaten aufgenommen wird. Denkbar ist des Weiteren, dass der zweite Sensor der Sensoranordnung eine Thermographie-Kamera ist, mit dem ein flächiges 2D-Thermographiebild als zweite Messdaten aufgenommen wird.

Vorteilhafterweise wird während des Vermessens der Fasermaterialoberfläche die Position und/oder Orientierung der Sensoranordnung erfasst, wobei das Fusionieren der Messdaten weiterhin in Abhängigkeit von der erfassten Position und/oder Orientierung erfolgt. So ist es denkbar, dass in diskreten Zeitabschnitten die entsprechende Orientierung und/oder Position der Sensoranordnung aus den Telemetriedaten der Robotersteuerung abgegriffen und in einem Datenspeicher hinterlegt werden, sodass zu jedem einzelnen Eintrag der Messdaten die Position und/oder Orientierung der Sensoranordnung zu einem späteren Zeitpunkt ermittelbar ist. Unter Kenntnis der Übertragungsfunktion wird es Möglichkeit, dass eine Sensorkoordinatensystem in das jeweils andere Sensorkoordinatensystem zu überführen. Aufgrund der Tatsache, dass die Sensoranordnung fest an einem Bewegungsautomaten angeordnet ist, lassen sich die Sensorkoordinatensysteme in das Referenzkoordinatensystem des Bewegungsautomaten überführen, sodass unter Zugrundelegung der Position und/oder Orientierung der Sensoranordnung definiert in Referenzkoordinatensystem des Bewegungsautomaten die Messdaten entsprechend fusioniert werden können.

Es ist darüber hinaus ebenfalls vorteilhaft und vom Kerngedanken her umfasst, wenn die Sensoranordnung in einem Nachlauf einer Faserlegeanlage angeordnet ist und dabei mit Hilfe der Sensoranordnung bzw. des ersten und des zweiten Sensors während des Ablegens von Fasermaterial die bereits abgelegten Fasermaterialien vermessen werden. Somit kann im bestehenden Inline Prozess eine Fehlererkennung durchgeführt werden, indem die fusionierten Messdaten hinsichtlich des Auftretens von Fehlstellen analysiert und untersucht werden.

Demzufolge ist ein weiterer Aspekt der vorliegenden Erfindung ein Verfahren zum Ablegen von Fasermaterial eines Faserverbundwerkstoffes mittels einer Faserlegeanlage, die einen Bewegungsautomaten und einen als Endeffektor an dem Bewegungsautomaten angeordneten Faserlegekopf hat, wobei an dem Faserlegekopf eine Sensoranordnung angeordnet ist, die einen ersten Sensor einer ersten Sensorart zum Erfassen einer ersten Merkmalsart der Fasermaterialoberfläche und wenigstens einen zweiten Sensor einer zweiten Sensorart zum Erfassen einer von der ersten Merkmalsart verschiedenen zweiten Merkmalsart der Fasermaterialoberfläche hat, wobei das Verfahren folgende Schritte umfasst:
- Kalibrieren der Sensoranordnung mit dem zuvor beschriebenen Verfahren;
- Ablegen des Fasermaterials mit Hilfe des Faserlegkopfes, wobei während des Ablegens des Fasermaterials die Fasermaterialoberfläche bereits abgelegter Fasermaterialien mittels der Sensoranordnung vermessen wird, wobei während des Vermessens erste Messdaten durch den ersten Sensor und zweite Messdaten durch den zweiten Sensor ermittelt werden,
- Fusionieren der ersten Messdaten mit den zweiten Messdaten unter Berücksichtigung der bei der Kalibrierung ermittelten Übertragungsfunktion zu einem fusionierten Messdatensatz, und
- Untersuchen des fusionierten Messdatensatzes zum Erkennen von Fehlstellen auf der Fasermaterialoberfläche mittels einer Fehlererkennungseinheit.

Die Erfindung wird an beigefügten Figuren beispielhaft erläutert:
- Figur 1 :: schematische Abbildung der relevanten Koordinatensysteme des Gesamtsystems;
- Figur 2:: schematische Darstellung eines Kalibrierkörpers;
- Figur 3:: schematische Darstellung der Überlagerung der Messdaten;
- Figur 4:: schematische Darstellung der auftretenden und zu kompensierenden Verzerrungen eines Messbildes.

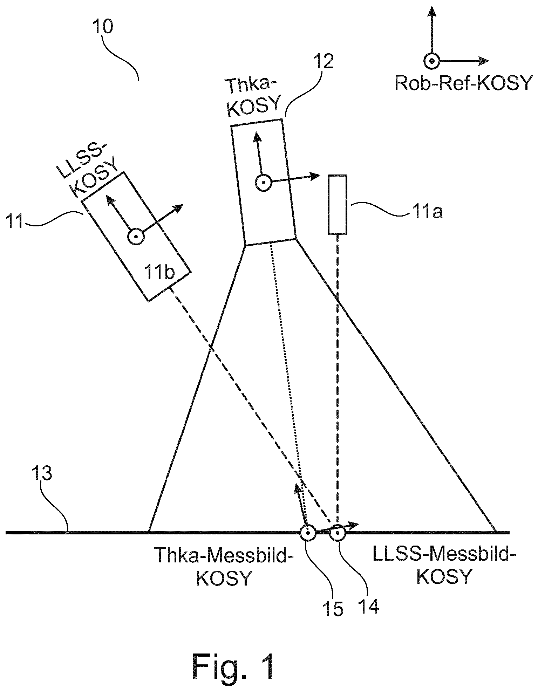

Figur 1 zeigt schematisch die relevanten Koordinatensysteme des Gesamtsystems. Eine Sensoranordnung 10 weist dabei einen ersten Sensor 11 sowie einen zweiten Sensor 12 auf, wobei die Sensoranordnung 10 in Richtung einer zu vermessenden Fasermaterialoberfläche 13 ausgerichtet ist. Die Fasermaterialoberfläche 13 ist dabei der relevante Betrachtungsbereich der Sensoranordnung 10.

Der erste Sensor 11 ist dabei ein Lichtschnittsensor, bei dem mit Hilfe einer Laserquelle 11a ein Laserstrahl auf die Fasermaterialoberfläche 13 an einem ersten Punkt 14 projiziert wird, wobei mit Hilfe eines beabstandeten Kamerasystems 11b die auf die Fasermaterialoberfläche 13 im ersten Punkt 14 projizierte Laserlinie unter einem Winkel aufgenommen wird. Höhenunterschiede im ersten Punkt 14 der Laserlinie der Laserquelle 11a werden durch Verzerrung der Laserlinie durch das Kamerasystem 11b aufgezeichnet.

Der zweite Sensor ist eine Thermographie-Kamera, deren Orientierung im zweiten Punkt 15 der Fasermaterialoberfläche 13 ausgerichtet ist.

Die Sensoranordnung 10 ist dabei an einem nichtdargestellten Bewegungsautomaten bzw. Roboter angeordnet, sodass die Sensoranordnung 10 hinsichtlich ihrer Position und Orientierung im Raum frei bewegbar ist. Das als Rob-Ref-KOSY bezeichnete Roboter-Referenz-Koordinatensystem stellt dabei die Referenz dar, in deren Bezug sich die Position und Anordnung der Sensoranordnung 10 als auch der Betrachtungsbereich bzw. die Fasermaterialoberfläche 13 befinden.

Hiervon verschieden sind das Koordinatensystem des Laserlichtschnittsensors (LLSS-KOSY) und das Koordinatensystem der Thermographie-Kamera (Thka-KOSY) wodurch sich zwei unterschiedliche Messbildkoordinatensysteme auf der Fasermaterialoberfläche 13 im ersten Punkt 14 und im zweiten Punkt 15 ergeben. Diese verschiedenen Koordinatensysteme sind dabei als LLSS-Messbild-KOSY und Thka-Messbild-KOSY bezeichnet.

Der Laserlichtschnittsensor 11 zeichnet ein Höhenprofil der Fasermaterialoberfläche 13 auf, das aus einzelnen sogenannten Profiles zusammengesetzt ist. Diese einzelnen Profiles werden in diskreten und vorab definierten Abständen aufgenommen. Die rückprojizierte Laserlinie des Laserlichtschnittsensors kann dabei mit einem flächigen Sensor oder einem Zeilensensor bzw. einer Zeilenkamera aufgenommen werden. Allerdings stellen immer nur wenige, durch die laserbeleuchteten Bildpunkte relevante Informationen bereit, wodurch der Laserlichtschnittsensor zum Aufzeichnen des Höhenprofils an einem Zeilensensor angelehnt ist.

Die Aufzeichnung eines Laserlichtschnittsensor-Messdatensatzes erfolgt durch einen Verfahrvorgang des Laserlichtschnittsensors über dem Betrachtungsbereich. Hierbei werden in diskreten Abständen die beschriebenen Profiles aufgezeichnet. Ein Laserlichtschnittsensor-Messdatensatz kann dabei neben Tiefeninformationen auch Rückstrahl- und Linienbreiteninformationen enthalten. Diese werden für jedes einzelne aufgezeichnete Profile ermittelt und abgelegt.

Bei der als zweiten Sensor verwendeten Thermographie-Kamera kann es speziell bei preisgünstigen Thermographie-Kameras bedingt durch ihr Funktionsprinzip Limitationen in der Aufnahmegeschwindigkeit einzelner Messbilder geben. Im hier beschriebenen Anwendungsfall wird ein 2D-Thermographiebild des Messbereiches erzeugt, wobei diese Bildaufnahme während der Aufzeichnung der einzelnen Höhenprofile im Lichtschnittsensor, bspw. etwa mittig über dem Messbereich, erfolgt. Durch die Limitationen der Thermographie-Kamera können die Aufnahmezeitpunkte und damit verbunden die Aufnahmepositionen des Thermographie-Messbildes nur in eingeschränktem Rahmen vorgegeben werden. Im hier beschriebenen Anwendungsfall beträgt die Latenz zwischen Auslösen und Bildaufnahme bis zu 1/30 Sekunden.

Es ist somit erforderlich, die Messpunkte des einzeln erzeugten Thermographiekamera-Messbildes auf die kontinuierlich erzeugten Messpunkte des Laserlichtschnittsensors zurückzuführen. Es muss eine Übertragungsfunktion für das Thermographiekamera-Messbild-Koordinatensystem gegenüber dem Laserlichtschnittsensor-Messbild-Koordinatensystem bestimmt werden, sodass die durch einen solchen mathematischen Zusammenhang fusionierbaren Sensordaten durch die vorgeschaltete Kalibrierung des Laserlichtschnittsensors somit auch in Bezug zum Roboter-Koordinatensystem (Rob-Ref-KOSY) gesetzt werden. Die so fusionierten Sensordaten sind damit vollumfänglich und als Datenkombination nutzbar.

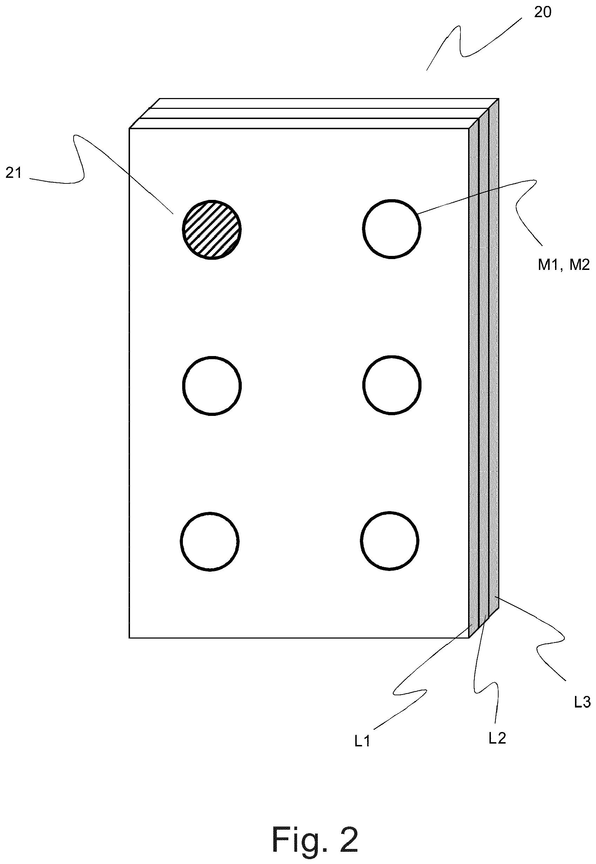

Figur 2 zeigt beispielhaft einen Kalibrierkörper 20, der aus insgesamt drei verschiedenen Lagen schichtweise aufgebaut ist. Die oberste Lage L1 besteht dabei aus einem wärmeleitenden Material, in welches in einer regelmäßigen Struktur kreisrunde Löcher 21 eingebracht sind. Eine möglichst homogene Anordnung dieser Löcher 21 ist dabei vorteilhaft. Die unterste Lage L3 des Kalibrierkörpers 20 besteht ebenfalls aus wärmeleitendem Material. Diese Lage ist an den offenen Löchern 21 der ersten Lage L1 jedoch geschlossen. Die mittlere Lage L2 besteht aus einer wärmeisolierenden Schicht. Aussparungen in der obersten Lage L1 sind hier entsprechend berücksichtigt und ausgeschnitten, sodass die Löcher 21 des Kalibrierkörpers 20 auf der untersten Lage L3 enden. Die obere Lage L1 und untere Lage L3 strahlen IR-Strahlungen mit unterschiedlicher Intensität ab. Dieses Verhalten kann z. B. durch Erwärmung einer der beiden Lagen (bspw. die unterste Lage L3) aber auch durch das aktive Leuchten eine dieser Schichten im IR-Spektralbereich realisiert werden. Zur besseren visuellen Abgrenzung bzw. Einprägung eines abweichenden Reflektionsverhaltens können die obere und die untere Lage auch in unterschiedlichen Kontrasten ausgeführt sein.

Die Löcher 21 des Kalibrierkörpers 20 stellen demzufolge Merkmale dar, die von dem zweiten Sensor, nämlich der Thermographiekamera, erfasst werden können. Demzufolge handelt es sich bei den Löchern 21 um durch den zweiten Sensor erfassbare zweite Merkmale M2. Gleichzeitig sind diese Löcher jedoch auch in einem Höhenprofil erkennbar, sodass es sich bei diesen Löchern 21 auch um Abweichungen von dem normalen Höhenprofil des Kalibrierkörpers 20 handelt und somit um durch den ersten Sensor erkennbare erste Merkmale M1.

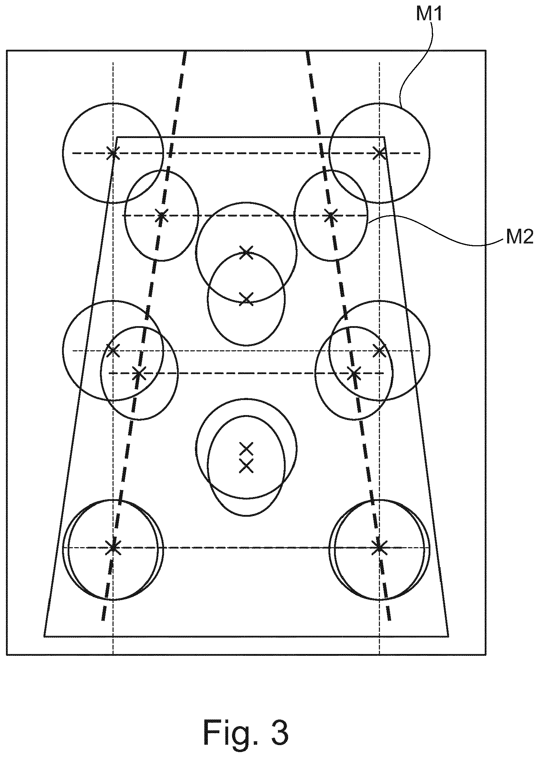

Figur 3 zeigt schematisch die Überlagerung des Messbildes des Laserlichtschnittsensors mit dem Messbild der Thermographiekamera, wie sie bei der Vermessung des Kalibrierkörpers entstehen. Wie zu erkennen ist, sind die beiden Messbilder aufgrund der abweichenden Koordinatensysteme der Sensoren nicht deckungsgleich, sodass nun eine Übertragungsfunktion ermittelt werden muss, um eines oder beide Messbilder so zu manipulieren, dass die Bilder deckungsgleich werden.

Hierdurch werden Messpunkte des einen Messbildes den Messpunkten des jeweils anderen Messbildes zugeordnet. Die in Figur 3 skizzierten Löcher stellen dabei jeweils die für das jeweilige Messbild erkennbaren Merkmale dar, wobei im unteren Messbild das Merkmal M1 durch den ersten Sensor (Laserlichtschnittsensor) erfasst wurde, während das darüber liegende verzerrte Messbild durch die Thermographiekamera aufgenommen wurde und dabei ein Merkmal M2 enthält, dass nun in Deckung gebracht werden muss mit dem Merkmal M1.

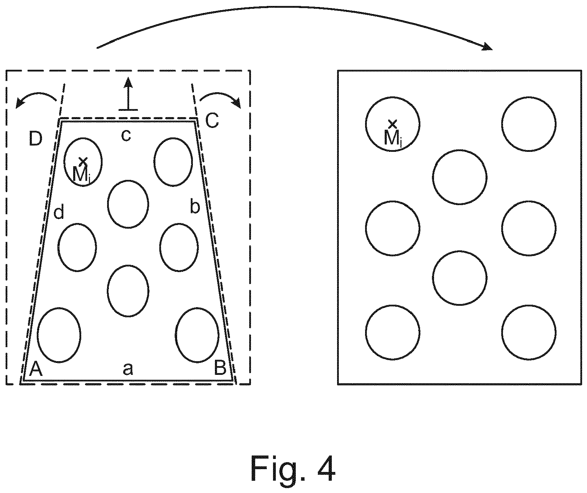

Bei der Ermittlung der Übertragungsfunktion mit dem oben beschriebenen Kalibriermuster wird das Thermographiekamera-Messbild auf das Laserlichtschnittsensor-Messbild gemappt, wobei hierzu ein Rotationsvektor R, ein Translationsvektor T und ein Verzerrungsvektor D ermittelt wird. Dies ist schematisch in Figur 4 gezeigt. Der Vektor R rotiert das gesamte Bild, der Vektor T verschiebt das Bild translatorisch und der Vektor D verzerrt das Bild dahingehend trapezförmig, dass die perspektivische Verzerrung des Thermographiekamera-Messbildes gegenüber dem Laserlichtschnittsensor-Messbild bestenfalls eliminiert, aber zumindest stark minimiert wird. Die Vektoren R und T besitzen jeweils zwei Einträge, welche die Rotation und Translation für jede Richtung des 2D-Bildes abbilden. Der Vektor D, zur Eliminierung der trapezförmigen Verzerrung, ist wie folgt aufgebaut:
D ist gleich (a_diff, β_diff, γ_difif, δ_diff)
oder
D ist gleich (a_diff, b_diff, c_diff, d_diff)

Der Vektor D beinhaltet jeweils 4 Einträge und kann einerseits die Differenz der Kantenwinkel zum gewünschten Verzerrungszustand beinhalten, oder aber die vektorielle Differenz der Kanten zum gewünschten Zielzustand. Für die vektorielle Betrachtung repräsentiert jeder Eintrag wiederum ein 2D-Vektor, welcher die Anpassung der einzelnen Kanten in vektorieller Form beschreibt (z. B *a_diff =* (*aₓ_diff,a_{y}_diff)).* Der Vektor D beschreibt somit ein neues Trapez, welches zur Kompensation der perspektivischen und im Bild trapezförmig manifestierten Verzerrung dient.

Die oben beschriebenen Vektoren können dabei mit Hilfe der Auswerteeinheit automatisiert erzeugt werden. Hierzu können die einzelnen Vektoren iterativ angepasst werden, sodass die einzelnen Messbilder möglichst gut überlappen. Aufgrund der getroffenen Annahmen und Randbedingungen genügt es in diesem Fall die durch den Mittelpunkt der einzelnen Kreise gebildeten Geraden des Kalibriermusters in jedem Messbild zu ermitteln und die entsprechende Differenz zueinander zu ermitteln. Entsprechend der Abweichung werden die Vektoren R, T und D zur Kompensation dieser Effekte entsprechend gewählt.

Aufgrund der stark schwankenden Auslöselatenz der Thermographiekamera ist gegebenenfalls eine Anpassung des Translationskorrekturvektors notwendig. Die mit dieser Auslöseverzögerung einhergehende translatorische Positionsänderung der Ist-Aufnahmeposition gegenüber der Soll-Aufnahmeposition ist hierbei gut messbar. Dies bedeutet, dass der Korrekturvektor für die Translation T entsprechend der Verfahrgeschwindigkeit in kleinem Maße während des Prozesses angepasst werden kann.

### Bezugszeichenliste

- 10: Sensoranordnung
- 11: Erster Sensor
- 11a: Laserquelle
- 11b: Kamerasystem
- 12: Zweiter Sensor
- 13: Fasermaterialoberfläche
- 14: Erster Punkt
- 15: Zweiter Punkt
- 20: Kalibrierkörper
- 21: Löcher
- L1: Erste Lage
- L2: Zweite Lage
- L3: Dritte Lage
- M1: erste Merkmale der erste Merkmalsart
- M2: zweite Merkmale der zweite Merkmalsart

## Patentansprüche

1. Verfahren zum Kalibrieren einer Sensoranordnung (10), die zum Ermitteln von fusionierten Messdaten einer Fasermaterialoberfläche (13) eines Fasermaterial eines Faserverbundwerkstoffes, aus dem ein Faserverbundbauteil herstellbar ist, vorgesehen ist, wobei die Sensoranordnung (10) einen ersten Sensor (11) einer ersten Sensorart zum Erfassen einer ersten Merkmalsart (M1) der Fasermaterialoberfläche (13) und wenigstens einen zweiten Sensor (12) einer zweiten Sensorart zum Erfassen einer von der ersten Merkmalsart (M1) verschiedenen zweiten Merkmalsart (M2) der Fasermaterialoberfläche (13) hat, wobei das Verfahren die folgenden Schritte umfasst:
- Bereitstellen eines Kalibrierkörpers (20), der Merkmale der ersten Merkmalsart (M1) und Merkmale der zweiten Merkmalsart (M2) aufweist;
- Ermitteln von ersten Messdaten durch Vermessen des Kalibrierkörpers (20) mittels des ersten Sensors (11) der Sensoranordnung (10) und Ermitteln von zweiten Messdaten durch Vermessen des Kalibrierkörpers (20) mittels des zweiten Sensors (12) der Sensoranordnung (10);
- Erkennen von ersten Merkmalen der ersten Merkmalsart (M1) aus den ersten Messdaten und Erkennen von zweiten Merkmalen der zweiten Merkmalsart (M2) aus den zweiten Messdaten mittels einer Auswerteeinheit;
- Vergleich der erkannten ersten Merkmale mit den erkannten zweiten Merkmalen bezogen auf die relative Position, Orientierung und/oder Form der Merkmale; und
- Ermitteln einer Übertragungsfunktion in Abhängigkeit von dem Vergleich der erkannten Merkmale, um einen jeweiligen Messwert der ersten Messdaten mit einem entsprechenden Messwert der zweiten Messdaten zu korrelieren.

2. Verfahren nach einem der vorhergehenden Ansprüche, **dadurch gekennzeichnet, dass** der erste Sensor (11) der Sensoranordnung (10) ein Laserlichtschnittsensor ist, mit dem zeilenweise ein Höhenprofil als erste Messdaten aufgenommen wird.

3. Verfahren nach Anspruch 2, **dadurch gekennzeichnet, dass** der Kalibrierkörper (20) derart bereitgestellt wird, dass auf der Oberfläche des Kalibrierkörpers (20) mindestens ein Bereich mit einem abweichenden Höhenniveau vorgesehen ist.

4. Verfahren nach einem der vorhergehenden Ansprüche, **dadurch gekennzeichnet, dass** der zweite Sensor (12) der Sensoranordnung (10) eine Thermografiekamera ist, mit dem ein flächiges 2D-Termographiebild als zweite Messdaten aufgenommen wird.

5. Verfahren nach Anspruch 4, **dadurch gekennzeichnet, dass** der Kalibrierkörper (20) derart bereitgestellt wird, dass an dem Kalibrierkörper (20) mindestens ein Bereich mit einer abweichenden thermographischen Rückstrahleigenschaft vorgesehen ist.

6. Verfahren nach Anspruch 3 und 5, **dadurch gekennzeichnet, dass** der mindestens eine Bereich mit dem abweichenden Höhenniveau dem Bereich mit der mindestens einen abweichenden thermographischen Rückstrahleigenschaft entspricht.

7. Verfahren nach einem der vorhergehenden Ansprüche, **dadurch gekennzeichnet, dass** in den ersten und/oder zweiten Messdaten intrinsische Sensorabweichungen durch die Auswerteeinheit eliminiert werden.

8. Verfahren nach einem der vorhergehenden Ansprüche, **dadurch gekennzeichnet, dass** die Übertragungsfunktion in Abhängigkeit von
- einer translatorischen Ablage der erkannten ersten Merkmale in Bezug zu den erkannten zweiten Merkmalen,
- einer rotatorischen Ablage der erkannten ersten Merkmale in Bezug zu den erkannten zweiten Merkmalen, und/oder
- einer perspektivischen Verzerrung der erkannten ersten Merkmale in Bezug zu den erkannten zweiten Merkmalen
ermittelt wird.

9. Verfahren zum Vermessen einer Fasermaterialoberfläche (13) eines Fasermaterials eines Faserverbundwerkstoffes, aus dem ein Faserverbundbauteil herstellbar ist, mittels einer Sensoranordnung (10), wobei die Sensoranordnung (10) einen ersten Sensor (11) einer ersten Sensorart zum Erfassen einer ersten Merkmalsart (M1) der Fasermaterialoberfläche (13) und wenigstens einen zweiten Sensor (12) einer zweiten Sensorart zum Erfassen einer von der ersten Merkmalsart (M1) verschiedenen zweiten Merkmalsart (M2) der Fasermaterialoberfläche (13) hat, wobei das Verfahren die folgenden Schritte umfasst:
- Kalibrieren der Sensoranordnung (10) mit dem Verfahren nach einem der vorhergehenden Ansprüche;
- Vermessen der Fasermaterialoberfläche (13) mittels der Sensoranordnung (10), wobei während des Vermessens erste Messdaten durch den ersten Sensor (11) und zweite Messdaten durch den zweiten Sensor (12) ermittelt werden, und
- Fusionieren der ersten Messdaten mit den zweiten Messdaten unter Berücksichtigung der bei der Kalibrierung ermittelten Übertragungsfunktion zu einem fusionierten Messdatensatz.

10. Verfahren nach Anspruch 9, **dadurch gekennzeichnet, dass** mittels einer Fehlererkennungseinheit der fusionierte Messdatensatz zum Erkennen von Fehlstellen auf der Fasermaterialoberfläche (13) untersucht wird.

11. Verfahren nach Anspruch 9 oder 10, **dadurch gekennzeichnet, dass** der erste Sensor (11) der Sensoranordnung (10) ein Laserlichtschnittsensor ist, mit dem zeilenweise ein Höhenprofil als erste Messdaten aufgenommen wird.

12. Verfahren nach einem der Ansprüche 9 bis 11, **dadurch gekennzeichnet, dass** der zweite Sensor (12) der Sensoranordnung (10) eine Thermografiekamera ist, mit dem ein flächiges 2D-Termographiebild als zweite Messdaten aufgenommen wird.

13. Verfahren nach einem der Ansprüche 9 bis 12, **dadurch gekennzeichnet, dass** während des Vermessens der Fasermaterialoberfläche (13) die Positionen und/oder Orientierungen der Sensoranordnung (10) erfasst, wobei das Fusionieren der Messdaten weiterhin in Abhängigkeit von den erfassten Positionen und/oder Orientierungen erfolgt.

14. Vorrichtung zum Vermessen einer Fasermaterialoberfläche (13) eines Fasermaterials eines Faserverbundwerkstoffes, aus dem ein Faserverbundbauteil herstellbar ist, mittels einer Sensoranordnung (10), wobei die Sensoranordnung (10) einen ersten Sensor (11) einer ersten Sensorart zum Erfassen einer ersten Merkmalsart der Fasermaterialoberfläche (13) und wenigstens einen zweiten Sensor (12) einer zweiten Sensorart zum Erfassen einer von der ersten Merkmalsart verschiedenen zweiten Merkmalsart der Fasermaterialoberfläche (13) hat, **dadurch gekennzeichnet, dass** die Vorrichtung zur Durchführung des Verfahrens nach einem der Ansprüche 9 bis 13 eingerichtet ist.
